# EUROPÄISCHE PATENTSCHRIFT

(11) **EP 1 092 703 B1**
(45) Veröffentlichungstag und Bekanntmachung des Hinweises auf die Patenterteilung: **07.01.2004**
(21) Anmeldenummer: 00121716.5
(22) Anmeldetag: 05.10.2000
(51) Int. Cl.: C07C 67/03, C07C 69/52, C07C 69/24

(54) **Verfahren zur Herstellung von Fettsäuremethylestern**
Process for the preparation of methyl esters of fatty acids
Procédé de préparation d'esters méthyliques d'acides gras

(30) Priorität: 14.10.1999 DE 19949718
(43) Veröffentlichungstag der Anmeldung: 18.04.2001
(73) Patentinhaber: Cognis Deutschland GmbH & Co. KG, 40589 Düsseldorf (DE)
(72) Erfinder: Büttgen, Karl-Heinz, 50170 Kerpen (DE); Lindemann, Manfred, Dr., 42719 Solingen (DE); Kubersky, Hans Peter, Dr., 42651 Solingen (DE); Wollmann, Gerhard, Dr., 40723 Hilden (DE); Falkowski, Jürgen, 40627 Düsseldorf (DE)

(56) Entgegenhaltungen:
- EP-A- 0 391 485

## Beschreibung

### Gebiet der Erfindung

Die Erfindung befindet sich auf dem Gebiet der oleochemischen Grundstoffe und betrifft ein Verfahren zur Herstellung von Fettsäuremethylestern durch Kombination von Hockdruck- und Niederdruckumesterung ("Hybridfahrweise") von Glyceriden.

### Stand der Technik

Fettsäuremethylester stellen wichtige Grundstoffe der Oleochemie dar und werden überwiegend zu den entsprechenden Fettalkoholen hydriert. Zu ihrer Herstellung geht man von Glyceriden, vorzugsweise natürlichen Fetten und Ölen aus, die entweder einer Hoch- oder Niederdruckumesterung unterworfen werden, d.h. in den Estern wird der Glycerinanteil gegen Methanol ausgetauscht. Das freigesetzte Glycerin stellt dabei ein wertvolles Koppelprodukt dar, das bei entsprechender Reinheit ebenfalls vielfältige Anwendungen, beispielsweise in Kosmetik und Pharmazie findet.

Die Hochdruckumesterung von Triglyceriden ist seit langem großtechnisch realisiert. In Gegenwart von Zinkseifen werden die Fette und Öle bei Temperaturen von typischerweise 230 °C und Drücken im Bereich von 50 bis 100 bar zu den Methylestern umgesetzt. Das Verfahren erlaubt den Einsatz auch unraffinierter, d.h. saurer Einsatzstoffe (Säurezahlen bis etwa 20) und liefert ein praktisch salzfreies Glycerin. Von Nachteil ist jedoch, daß das freigesetzte Glycerin nicht ohne weiteres dem Estergleichgewicht entzogen werden kann, um die Reaktion auf die Seite der Produkte zu verlagern. Zu diesem Zweck muß vielmehr ein hoher Überschuß an Methanol eingesetzt werden, was in der Folge mit hohen Energiekosten verbunden ist, da nicht umgesetzter Alkohol am Ende der Reaktion wieder abgetrennt und wieder aufbereitet werden muß. Dennoch liegt der Umsatz beim Hochdruckverfahren in der Regel nur in der Gegend von 92 %, so daß eine hohe Rückstandsmenge anfällt. Bei der Destillation der Reaktionsmischung kommt es femer zu einer partiellen Rückveresterung in Glyceride und freies Glycerin, welche dann in der Hydrierung der Ester zu den Alkoholen stören. Schließlich werden in der Umesterung hohe Temperaturen und hohe Katalysatorkonzentration benötigt, um eine hinreichend niedrige Verweilzeit sicherzustellen.

Eine Alternative hierzu bietet das vor einigen Jahren entwickelte Niederdruckumesterungsverfahren, das beispielsweise Gegenstand der europäischen Patentschrift **EP 0494177 B1** (Henkel) ist Hierbei werden die Glyceride bei Temperaturen um 80 °C und Drücken von 1 bis 1,2 bar umgeestert. Die Reaktion wird in Gegenwart von Alkalibasen, vorzugsweise Natriummethylat in zwei Stufen durchgeführt. Nach Durchlaufen des ersten Reaktors wird die Reaktionsmischung über einen Schwerkrafttrenner vom Glycerin befreit, zur Verlagerung des Reaktionsgleichgewichtes wird also im Gegensatz zum Hochdruckverfahren kein so erheblicher Methanolüberschuß benötigt Dies verbunden mit einem höheren Umsatz von etwa 97 % und den deutlich milderen Bedingungen stellt aus ökonomischer Sicht eine erhebliche Verbesserung dar. Nichtsdestotrotz kann auch das Niederdruckverfahren nicht völlig befriedigen, da auch ihm Nachteile anhaften. Werden nämlich saure Einsatzstoffe verwendet, wird ein Teil des Alkalikatalysators desaktiviert, d.h. als Salz gebunden. Demzufolge steigt mit der Säurezahl der eingesetzten Triglyceride die Menge an Katalysator und vor allem der Salzgehalt des gebildeten Glycerins. Der Einsatz beispielsweise von nicht raffiniertem Kokosöl mit einer Säurezahl von etwa 10, der aus ökonomischen Gründen sehr bevorzugt ist, scheidet daher für eine Niederdruckumesterung aus.

Demzufolge hat die Aufgabe der vorliegenden Erfindung darin bestanden, ein Verfahren zur Herstellung von Fettsäuremethylestern mit möglichst niedriger Säurezahl zur Verfügung zu stellen, das die Nachteile der Hoch- und Niederdruckumesterung vermeidet, insbesondere aber den Einsatz saurer, nicht raffinierter Einsatzstoffe erlaubt und als Koppelprodukt ein Glycerin liefert, welches einen Salzgehalt von weniger als 1 Gew.-% aufweist

### Beschreibung der Erfindung

Gegenstand der Erfindung ist ein Verfahren zur Herstellung von Fettsäuremethylestern mit Säurezahlen kleiner 0,1, welches sich dadurch auszeichnet, daß man
(a) Fettsäureglyceride mit Säurezahlen im Bereich von 5 bis 20 mit Methanol im Einsatzverhältnis 0,4 bis 0,7 Volumenprozent und in Gegenwart einer wirksamen Menge einer Übergangsmetallseife einer ersten Hochdruckumesterung bei Temaeraturen im Bereich von 150 bis 250 °C und Drücken im Bereich von 50 bis 150 bar unterwirft und dabei ein Zwischenprodukt herstellt, welches eine Säurezahl von maximal 0,75, vorzugsweise unter 0,5 aufweist,
(b) das Zwischenprodukt entspannt und das Glycerin mit Hilfe eines Abscheiders abtrennt und
(c) das von Glycerin befreite Zwischenprodukt unter Zugabe einer wirksamen Menge einer Alkalibase einer zweiten Stufe einer Niederdruckumesterung bei Temperaturen im Bereich von 50 bis 100 °C und Drücken im Bereich von 1 bis 5 bar unterwirft

Überraschenderweise wurde gefunden, daß durch Kombination von Hochdruck- und Niederdrukkumesterung ("Hybridfahrweise") in der oben beschriebenen Weise auch auf Basis saurer Ausgangsstoffe Fettsäuremethylester mit Säurezahlen von kleiner 0,1 und typisch 0,01 erhalten werden. Gleichzeitig weist das als Koppelprodukt erhaltene Glycerin einen Salzgehalt von kleiner 1 und typisch 0,6 Gew.-% auf. Das Verfahren minimiert gegenüber dem Hochdruckverfahren zudem die Methanol- und Rückstandsmenge ganz erheblich. Des weiteren findet in der Destillation praktisch keine Rückveresterung mehr statt.

### Fettsäureglyceride

Die Auswahl der als Einsatzstoffe in Betracht kommenden Fettsäureglyceride ist an sich wenig kritisch. Vorzugsweise folgen sie der Formel **(I),**

**R**^{**1**}**CO-OCH**_{**2**}**CH(OR**^{**2**}**)CH**_{**2**}**OR**^{**3**} **(I)**

in der R¹CO für einen linearen oder verzweigten Acylrest mit 6 bis 24 Kohlenstoffatomen und 0 und/oder 1 bis 5 Doppelbindungen sowie R² und R³ unabhängig voneinander für Wasserstoff oder einen Acylrest R⁴CO mit 6 bis 24 Kohlenstoffatomen und 0 und/oder 1 bis 3 Doppelbindungen stehen, d.h. es kann sich um Mono-, Di- und/oder Triglyceride handeln. Die Glyceride können synthetischer Natur sein, vorzugsweise handelt es sich jedoch um natürliche Fette und Öle, wie z.B. Kokosöl, Palmöl, Palmkernöl, Olivenöl, Olivenkernöl, Rapsöl und Sonnenblumenöl, beide nach alter oder neuer Züchtung, Erdnußöl, Sesamöl, Reisöl, Babassuöl, Korianderöl, Meadowfoamöl, Leinöl, Rindertalg, Schweineschmalz und Fischöl. Das Ziel der vorliegenden Erfindung besteht darin, den Einsatz saurer, d.h. nicht raffinierter und damit besonders billiger Rohstoffe zu ermöglichen, es können natürlich auch entsäuerte Einsatzstoffe verwendet werden. Vorzugsweise setzt man als Fettsäureglyceride jedoch nicht raffinierte Kokosöle, Palmöle und/oder Palmkemöle ein, die insbesondere Säurezahlen im Bereich von 5 bis 15 aufweisen.

### Hochdruckumesterung

Im ersten Verfahrensschritt, bei dem etwa 85 bis 90 % der Einsatzstoffe umgesetzt werden, wird eine Hochdruckumesterung durchgeführt, die bei Temperaturen im Bereich von 150 bis 250, vorzugsweise 200 bis 230 °C und Drücken im Bereich von 50 bis 150, vorzugsweise 90 bis 110 bar stattfindet. Das molare Einsatzverhältnis zwischen Öl und Methanol liegt bei 0,4 bis 0,7 und vorzugsweise bei 0,5 bis 0,6. Als Katalysatoren kommen Übergangsmetallseifen in Frage, beispielsweise Zink- oder insbesondere Manganseifen, d.h. Salze der Palmitin- und/oder Stearinsäure. Die Katalysatormenge liegt - bezogen auf die Einsatzstoffe - typischerweise bei 0,01 bis 0,05 und vorzugsweise 0,02 bis 0,03 Gew.-%. Üblicherweise arbeitet man bei der Hochdruckumesterung im Gleichstromverfahren. Nach Verlassen des Reaktors wird die Mischung entspannt und in einen Separator geleitet, in dem eine Trennung zwischen der Glycerinphase und dem organischen Wertprodukt stattfindet. Letzteres wird dann zur weiteren Umsetzung einem zweiten Reaktor zugeleitet, in dem die Niederdruckumesterung erfolgt

### Niederdruckumesterung

Die Niederdruckumesterung wird bei Temperaturen im Bereich von 50 bis 100, vorzugsweise 70 bis 90 °C und Drücken im Bereich von 1 bis 5, vorzugsweise 1 bis 1,2 bar durchgeführt Eine Zudosierung von weiterem Methanol ist in der Regel nicht erforderlich. In manchen Fällen hat es sich sogar als nützlich erwiesen, einen Teil des Methanols, gegebenenfalls als Azeotrop mit Wasser, vor der Niederdruckumesterung in einer separaten Kolonne abzutreiben. Da die Zinkseifen bei so niedrigen Temperaturen nicht aktiv sind, setzt man der Reaktionsmischung in der Regel 0,01 bis 0,5, vorzugsweise 0,02 bis 0,05 Gew.-% einer Alkalibase zu. Hierfür eignen sich beispielsweise Natriumhydroxid, Kaliumhydroxid, Kalium-tert.butylat und insbesondere Natriummethylat Da das Zwischenprodukt bereits eine Säurezahl von kleiner 0,5 erreicht hat, gehen nur noch sehr kleine Mengen Katalysator durch Neutralisation verloren.

### Beispiele

**Beispiel 1.** Nicht raffiniertes Kokosöl (SZ = 9,5) wurde mit Methanol nach verschiedenen Verfahren umgeestert: Hybridverfahren (erfindungsgemäß, Beispiel 1) Hochdruckverfahren (Vergleichsbeispiel V1) und Niederdruckverfahren (Vergleichsbeispiel V2). Die Reaktionsbedingungen und Ergebnisse sind in Tabelle 1 zusammengefaßt. Nach dem Hochdruckverfahren wird zwar ein salzfreies Glycerin erhalten, der Umsatz ist jedoch niedrig. Das Niederdruckverfahren liefert umgekehrt hohe Umsätze, das Glycerin ist jedoch stark verunreinigt.

**Tabelle 1**

| Reaktionsbedingungen und Ergebnisse der Umesterung | | | |
|---|---|---|---|
| | **1** | **V1** | **V2** |
| ***Umesterungsreaktor I*** | | | |
| - Volumeneinsatzverhältnis Öl : Methanol | 0,63 | 1,0 | 0,28 |
| - Temperatur [°C] | 220 | 220 | 85 |
| - Druck [bar] | 75 | 75 | 3 |
| - Katalysator | StMn* | StMn | NaOMe |
| - Katalysatorkonzentration [Gew.-%] | 1,7 | 1,7 | 1,1 |
| - Verweilzeit [min] | 60 | 60 | 3 |
| ***Glycerinabscheidung*** | ja | nein | ja |

| ***Umesterungsreaktor II*** | | | |
|---|---|---|---|
| - Temperatur [°C] | 80 | 220 | 80 |
| - Druck [bar] | 1,2 | 110 | 3 |
| - Katalysator | NaOMe | StMn | NaOMe |
| - Katalysatorkonzentration [Gew.-%] | 0,3 | 0,22 | 0,06 |
| - Verweilzeit [min] | 3 | 60 | 3 |

| ***Produktzusammensetzung*** | | | |
|---|---|---|---|
| SZ Umesterungsprodukt nach Stufe 1 | 0,71 | 1,2 | < 0,1 |
| SZ Umesterungsprodukt nach Stufe 2 | < 0,1 | 0,9 | < 0,1 |
| Umsatz [% der Theorie] | 97 | 92 | 97 |
| Salzgehalt im Glycerin [Gew.-%] | 0,6 | < 0,1 | 11,3 |

| | | | |
|---|---|---|---|
| *) StMn = Manganstearat | | | |

## Patentansprüche

1. Verfahren zur Herstellung von Fettsäuremethylestern mit Säurezahlen kleiner 0,1 **dadurch gekennzeichnet, daß** man
(a) Fettsäureglyceride mit Säurezahlen im Bereich von 5 bis 20 mit Methanol im Einsatzverhältnis 0,4 bis 0,7 Volumenprozent und in Gegenwart einer wirksamen Menge einer Übergangsmetallseife einer ersten Hochdruckumesterung bei Temperaturen im Bereich von 150 bis 250°C und Drücken im Bereich von 50 bis 150 bar unterwirft und dabei ein Zwischenprodukt herstellt, welches eine Säurezahl von maximal 0,75 aufweist,
(b) das Zwischenprodukt entspannt und das Glycerin mit Hilfe eines Abscheiders abtrennt, und
(c) das von Glycerin befreite Zwischenprodukt unter Zugabe einer wirksamen Menge einer Alkalibase einer zweiten Stufe einer Niederdruckumesterung bei Temperaturen im Bereich von 50 bis 100°c und Drücken im Bereich von 1 bis 5 bar unterwirft.

2. Verfahren nach Anspruch 1, **dadurch gekennzeichnet, daß** man Fettsäureglyceride der Formel (I) einsetzt,
**R1CO-OCH2CH(OR2)CH2OR3 (I)**
in der R1CO für einen linearen oder verzweigten Acylrest mit 6 bis 24 Kohlenstoffatomen und 0 und/oder 1 bis 5 Doppelbindungen sowie R2 und R3 unabhängig voneinander für Wasserstoff oder einen Acylrest R4CO mit 6 bis 24 Kohlenstoffatomen und 0 und/oder 1 bis 3 Doppelbindungen stehen.

3. Verfahren nach den Ansprüchen 1 und/oder 2, **dadurch gekennzeichnet, daß** man als Fettsäureglyceride nicht raffinierte Kokosöle, Palmöle und/oder Palmkernöle einsetzt.

4. Verfahren nach mindestens einem der Ansprüche 1 bis 3, **dadurch gekennzeichnet, daß** man die Hochdruckumesterung in Gegenwart von - bezogen auf die Einsatzstoffe - 0,01 bis 0,05 Gew.-% einer Übergangsmetallseife durchführt.

5. Verfahren nach mindestens einem der Ansprüche 1 bis 4, **dadurch gekennzeichnet, daß** man die Niederdruckumesterung in Gegenwart von 0,01 bis 0,5 Gew.-% einer Alkalibase durchführt.

6. Verfahren nach mindestens einem der Ansprüche 1 bis 5, **dadurch gekennzeichnet, daß** man einen Teil des nicht umgesetzten Methanols, gegebenenfalls als Azeotrop, vor der Niederdruck-umesterung abtreibt.

## Claims

1. A process for the production of fatty acid methyl esters with acid values below 0.1, **characterized in that**
(a) fatty acid glycerides with acid values of 5 to 20 are subjected to a first highpressure transesterification with methanol in a ratio of 0.4 to 0.7% by volume at temperatures of 150 to 250°C, under pressures of 50 to 150 bar and in the presence of an effective quantity of a transition metal soap to form an intermediate product with an acid value of at most 0.75,
(b) the intermediate product is relieved of pressure and the glycerol is removed by means of a separator and
(c) the intermediate product freed from glycerol is subjected in a second stage to low-pressure transesterification at temperatures of 50 to 100°C, under pressures of 1 to 5 bar and in the presence of an effective quantity of an alkali metal base.

2. A process as claimed in claim 1, **characterized in that** fatty acid glycerides corresponding to formula (I):
**R**^{**1**}**CO-OCH**_{**2**}**CH(OR**^{**2**}**)CH**_{**2**}**OR**^{**3**} **(I)**
in which R¹CO is a linear or branched acyl group containing 6 to 24 carbon atoms and 0 and/or 1 to 5 double bonds and R² and R³ independently of one another represent hydrogen or an acyl group R⁴CO containing 6 to 24 carbon atoms and 0 and/or 1 to 3 double bonds, are used.

3. A process as claimed in claims 1 and/or 2, **characterized in that** unrefined coconut oils, palm oils and/or palm kernel oils are used as the fatty acid glycerides.

4. A process as claimed in at least one of claims 1 to 3, **characterized in that** the highpressure transesterification is carried out in the presence of 0.01 to 0.05% by weight, based on the starting materials, of a transition metal soap.

5. A process as claimed in at least one of claims 1 to 4, **characterized in that** the low-pressure transesterification is carried out in the presence of 0.01 to 0.5% by weight of an alkali metal base.

6. A process as claimed in at least one of claims 1 to 5, **characterized in that** part of the unreacted methanol is removed, optionally as an azeotrope, before the low pressure transesterification.

## Revendications

1. Procédé de préparation d'esters méthyliques d'acide gras ayant des indices d'acidité inférieurs à 0,1,
**caractérisé en ce qu'**
a) on soumet des glycérides d'acide gras ayant des indices d'acidité dans la zone de 5 à 20 à une première transestérification avec du méthanol dans un rapport d'utilisation de 0,4 à 0,7 % en volume et en présence d'une quantité efficace d'un savon de métal de transition, sous haute pression à des températures dans la plage de 150 à 250°C et à des pressions dans la plage de 50 à 150 bars et de cette façon on prépare un produit intermédiaire qui possède un indice d'acidité d'au maximum 0,75,
b) on détend le produit intermédiaire et on sépare le glycérol à l'aide d'un séparateur, et
c) on soumet le produit intermédiaire débarrassé du glycérol, sous addition d'une quantité efficace d'une base de métal alcalin, à une deuxième étape d'une transestérification à basse pression à des températures dans la plage de 50 à 100°C et à des pressions dans la plage de 1 à 5 bars.

2. Procédé selon la revendication 1,
**caractérisé en ce qu'**
on met en oeuvre des glycérides d'acides gras de formule (I),
R1CO-OCH2CH(OR2)CH2OR3 **(I)**
dans laquelle R1CO représente un reste acyle linéaire ou ramifié, ayant de 6 à 24 atomes de carbone et 0 et/ou 1 à 5 double liaisons ainsi que R2 et R3 indépendamment l'un de l'autre représentent de l'hydrogène ou un reste acyle R4CO ayant de 6 à 24 atomes de carbone et 0 et/ou 1 à 3 double liaisons.

3. Procédé selon l'une quelconque des revendications 1 ou 2,
**caractérisé en ce qu'**
on met en oeuvre comme glycéride d'acides gras, des huiles de coco, des huiles de palme et/ou des huiles de palmiste non raffinées.

4. Procédé selon au moins l'une quelconque des revendications 1 à 3,
**caractérisé en ce qu'**
on effectue la transestérification sous haute pression en présence de, rapporté aux substances utilisées, de 0,01 à 0,05 % en poids d'un savon de métal de transition.

5. Procédé selon au moins l'une quelconque des revendications 1 à 4,
**caractérisé en ce qu'**
on effectue la transestérification à basse pression en présence de 0,01 à 0,5 % en poids d'une base de métal alcalin.

6. Procédé selon au moins l'une quelconque des revendications 1 à 5,
**caractérisé en ce qu'**
on sépare une partie du méthanol qui n'a pas réagi, le cas échéant sous forme d'azéotrope, avant la transestérification à basse pression.
